# EUROPÄISCHE PATENTSCHRIFT

(11) **EP 1 740 722 B1**
(45) Veröffentlichungstag und Bekanntmachung des Hinweises auf die Patenterteilung: **06.08.2008**
(21) Anmeldenummer: 05740196.0
(22) Anmeldetag: 28.04.2005
(51) Int. Cl.: C12Q 1/68

(54) **VERFAHREN UND ANORDNUNG ZUR DNA-ISOLIERUNG MIT TROCKENREAGENZIEN**
METHOD AND ASSEMBLY FOR DNA ISOLATION WITH DRY REAGENTS
PROCEDE ET ENSEMBLE POUR L'ISOLEMENT D'ADN A L'AIDE DE REACTIFS SECS

(30) Priorität: 30.04.2004 DE 102004021780
(43) Veröffentlichungstag der Anmeldung: 10.01.2007
(73) Patentinhaber: SIEMENS AKTIENGESELLSCHAFT, 80333 München (DE)
(72) Erfinder: GUMBRECHT, Walter, 91074 Herzogenaurach (DE); PAULICKA, Peter, 91056 Erlangen (DE); STANZEL, Manfred, 91056 Erlangen (DE)
(86) Internationale Anmeldenummer: PCT/EP2005/051941
(87) Internationale Veröffentlichungsnummer: WO 2005/106024

(56) Entgegenhaltungen:
- WO-A-95/06652
- WO-A-99/33559
- WO-A-02/072262
- WO-A-20/04065010
- DE-A1- 10 236 460
- US-A1- 2001 012 612
- US-A1- 2002 022 261
- US-A1- 2002 045 246
- US-A1- 2002 106 686
- US-A1- 2003 073 110
- US-A1- 2004 018 611
- US-A1- 2004 063 197
- US-A1- 2004 063 198
- US-A1- 2004 209 354
- US-B1- 6 361 958
- MCMILLAN W A: "RAPID REAL-TIME PCR WITH FULLY INTEGRATED SPECIMEN PREPARATION" PHYTOPATHOLOGY, ST. PAUL, MN, US, Bd. 92, Nr. 6, SUPPL, 27. Juli 2002 (2002-07-27), Seite S110, XP008044514 ISSN: 0031-949X
- RAJA SIVA ET AL: "Technology for automated, rapid, and quantitative PCR or reverse transcription-PCR clinical testing." CLINICAL CHEMISTRY. MAY 2005, Bd. 51, Nr. 5, 3. März 2005 (2005-03-03), Seiten 882-890, XP001207245 ISSN: 0009-9147

## Beschreibung

Die Erfindung bezieht sich auf ein Verfahren zur DNA-Isolierung mit Trockenreagenzien. Daneben bezieht sich die Erfindung auch auf eine zugehörige Anordnung zur Durchführung des Verfahrens.

Bei der Nukleinsäureanalytik zur Beantwortung von humangenomischen Fragestellungen, z.B. in weißen Blutzellen aus Vollblut, müssen zunächst in einem Probenvorbereitungsschritt die Zellen aufgebrochen und anschließend die dabei freigesetzte DNA isoliert werden. Dabei müssen Blutbestandteile, wie z.B. Hämoglobin, Immunoglobuline und Laktoferrin, die eine nachfolgende PCR inhibieren könnten, entfernt werden.

Im Labor werden diese Arbeitsschritte nach hinlänglich bekanntem Stand der Technik durchgeführt. So können neben anderen Verfahren Zellen mit alkalischer Lösung (NaOH) aufgebrochen werden (sog. Lyse) und anschließend die DNA an mit Silica beschichteten Magnet-Beads gebunden werden. Durch Anlegen eines Magnetfeldes werden die mit DNA beladenen Magnet-Beads festgehalten und gewaschen. Die so isolierte DNA kann anschließend mit oder ohne Beads für eine PCR ("Polymerase Chain Reaction") eingesetzt werden.

Beim Stand der Technik werden die DNA-bindenden Magnet-Beads als Suspension in einem Zell-Lyse-Puffer eingesetzt. Alle Arbeitsschritte werden z.B. in 1,5 ml- Reaktionsgefäßen ( sog. "Eppendorf"-Gefäße) durchgeführt. Zu einem vorgegebenen Volumen der Bead-Suspension (z.B. 200 µl), wird z.B. 10 µl Vollblut zugegeben. Dabei werden die Blutzellen aufgeschlossen und die DNA freigesetzt. Die Magnet-Beads binden die DNA und bilden einen DNA/Bead-Komplex. Ein solcher DNA/Bead-Komplex kann anschließend durch ein Magnetfeld an der Gefäßwand des "Eppendorf-Tubes" fixiert, so dass Waschschritte zum Entfernen von PCR-inhibierenden Stoffen erfolgen können. Anschließend kann die PCR durchgeführt werden.

Die Realisierung letzteren Verfahrens ist vom Vorhandensein von Laborgeräten wie der "Eppendorf"-Gefäße (Tubes), Tube-Halte-Einrichtungen inkl. Magneten, Pipettier-Geräten, Kühlbehälter für Reagenzien abhängig und muss von geschultem Personal unter Einhaltung von Sicherheitsvorschriften (Infektionsgefahr, Abfallentsorgung, etc) durchgeführt werden. Mehrere volumetrische und hochgenaue Dosierungen von teilweise gesundheitsgefährdenden Stoffen (z.B. NaOH) müssen durch Pipettieren durchgeführt werden. Zusätzlich sind diese Arbeitsschritte zeitaufwendig.

Aus der US 2002/0022261 A1 sind bereits Systeme zur miniaturisierten genetischen Analyse und zugehörige Betriebsverfahren bekannt, bei dem eine Cartridge mit wenigstens einem Eingang und einer porösen Durchführung verwendet wird, die DNA-bindende Eigenschaften haben soll. Dabei erfolgt ein Zellaufschluss, wozu Reagenzien ggf. an der Gefäßwandung bereitgestellt werden. Weiterhin können strukturierte Bereiche der Wandung mit DNA-bindenden Materialien, gegebenenfalls auch Magnetbeads, belegt sein. Insgesamt ergeben sich dort eine Reihe von verschiedenen Vorschlägen zur Ausführung der Messung für die genetische Analyse, wobei aber noch kein durchgehendes Verfahren realisiert wird. Des Weiteren ist aus der EP 0 723 549 B1 ein Gemisch zum Isolieren von DNA bekannt, wobei speziell dieses Gemisch Silikagel, Glasteilchen und mindestens ein kaotropisches Salz enthält.

Weiterhin wird in der WO 99/33559 A eine Vorrichtung zur Trennung eines Analyten von einer Fluidprobe beschrieben, die eine Kartusche mit Lysierungskammer im Probenströmungsweg aufweist, wobei mittels eines Filters die Zellen der Probe herausgefiltert und aufgeschlossen werden. Der so separierte Analyt wird einzelnen Sensorkammern in der Vorrichtung zur Analyse zugeführt.

Von letzterem Stand der Technik ausgehend ist es Aufgabe der Erfindung, die DNA-Isolierung durchgängig in einer integrierten miniaturisierten Cartridge durchzuführen und eine zugehörige Anordnung zu schaffen.

Die Aufgabe ist erfindungsgemäß durch die Maßnahmen des Patentanspruches 1 gelöst. Eine zugehörige Anordnung ist im Patentanspruch 14 angegeben. Weiterbildungen des Verfahrens und der zugehörigen Anordnung sind Gegenstand der jeweiligen Unteransprüche.

Im Rahmen der Erfindung beinhaltet Patentanspruch 2 eine Ausbildung des Verfahrens mit einem Aufschluss von DNA enthaltenden biologischen Behältnissen, beispielsweise Zellen, Bakterien oder Viren. In besonders vorteilhafter und praxisgerechter Realisierung können somit Vollblutproben auf die DNA-Information untersucht werden.

Die Erfindung geht außer von obigem Stand der Technik insbesondere noch von der WO 02/072262 A1 mit der Bezeichnung "Analyseeinrichtung" aus. Dort werden trockengelagerte, bei Raumtemperatur stabile Reagenzien in Mikrokanälen bzw. Mikrokavitäten einer "Chipkarte" beschrieben, die durch Zuführen von Wasser, kurz vor Verwendung, in Lösung gebracht werden. Dabei wird darauf abgestellt, die Trockenreagenzien vordosiert bereitzustellen, so dass sich nach Auflösung ein quantitatives Analysemittel ergibt. Demgegenüber geht es bei vorliegender Erfindung um einen Zellaufschluss eines biologischen Behältnisses, beispielsweise die Isolierung von DNA aus einer Vollblut-Probe zwecks nachfolgender PCR und/oder Analyse, wobei die aus der Probe isolierten DNA geeigneter Weise bereitgestellt werden.

Mit dem erfindungsgemäßen Verfahren ergeben sich folgende Vorteile im Vergleich zur bisher angewandten Labormethode:
- Es erfolgt vollständige Integration aller Stoffe und Verfahren in einer geschlossenen einmalverwendbaren Cartridge;
- es ist eine Bevorratung der Reagenzien in einer sicheren, nicht gesundheitsgefährdenden, bei Raumtemperatur lagerstabilen Form gewährleistet;
- es sind keine manuellen Arbeitsschritte, außer der Injektion der Blutprobe, notwendig;
- es erfolgt kein direkter Kontakt mit gesundheitsgefährdenden Stoffen, d.h. Blut und Reagenzien-Abfall verbleiben in der Cartridge; die Cartridge ist klein und kostengünstig in Massenfertigung herzustellen.

Die erfindungsgemäße Anordnung weist folgende Merkmale auf:
- es ist mindestens ein Mikrokanal bzw. Mikrokavität vorhanden. Im Mikrokanal bzw. der Mikrokavität ist das Lyse-Mittel insbesondere unter Einschluss eines Filmbildners mit dem Substrat für die DNA an der Wandung des Kanals angebracht.

Ein in den Mikrokanal bzw. Mikrokavität eingebrachtes Lyse-Reagenz hat folgende Eigenschaften:
- Lyseeigenschaften für weiße Blutzellen und/oder andere Zellen, Bakterien, Viren;
- Feststoff, bzw. Flüssigkeit mit vernachlässigbarem Dampfdruck;
- stabil bei Raumtemperatur;
- gute Haftung an Mikrokanal- bzw. Mikrokavität-Wandungen.

Wesentlich ist bei der Erfindung, dass nunmehr in einer durchgängigen Verfahrenskette die in der Probe enthaltenden DNA freigesetzt, als isolierte DNA gesammelt und in geeigneter Weise zum Ort der PCR bzw. einer Detektion gebracht werden.

Weitere Einzelheiten und Vorteile der Erfindung ergeben sich aus der nachfolgenden Figurenbeschreibung von Ausführungsbeispielen anhand der Zeichnung in Verbindung mit den Patentansprüchen. Es zeigen
- Figur 1: die Draufsicht auf eine Analyseeinrichtung (Cartridge) und
- Figur 2 bis Figur 6: jeweils im Schnitt einen Ausschnitt aus Figur 1 längs der Linie II - II zur Verdeutlichung der DNA-Isolierung aus Vollblut, wobei die einzelnen Figuren 2 bis 6 jeweils unterschiedliche Funktions-stufen beinhalten.

In der nachfolgenden Beschreibung werden DNA enthaltene Proben definiert. Derartige Proben können Lösungen von DNA in einer Flüssigkeit, aber auch DNA enthaltende Suspensionen biologischer Behältnisse sein. Als biologische Behältnisse werden im vorliegenden Zusammenhang beispielsweise Zellen, Bakterien oder Viren verstanden. Zur Freisetzung der DNA ist dann ein Aufschluss des biologischen Behältnisses notwendig. Werden beispielsweise entsprechend einer humangenomischen Zielsetzung Vollblutproben verwendet, bedarf es eines Zellaufschlusses der weißen Blutzellen, in denen die DNA lokalisiert ist.

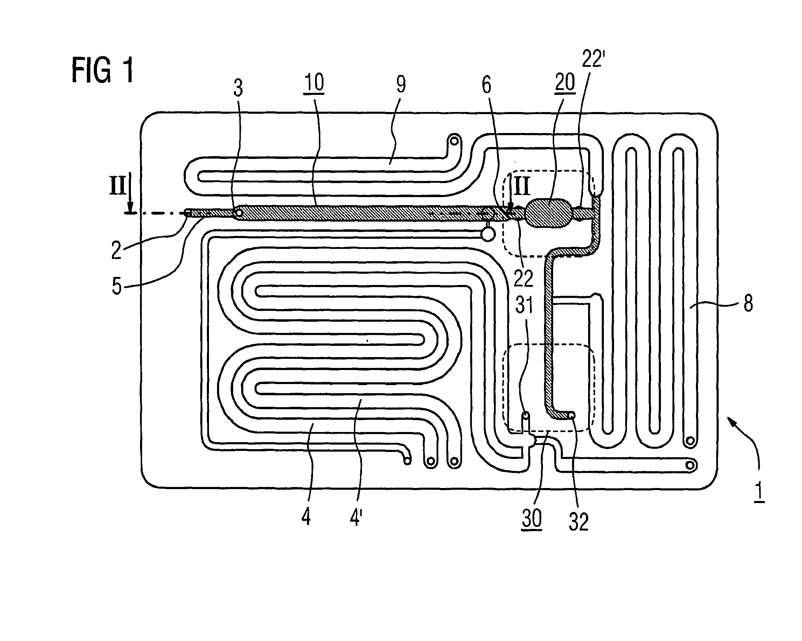

In Figur 1 ist eine Analyseeinrichtung dargestellt, die nachfolgend auch als "Cartridge" und als zentrale oder dezentrale Messeinrichtung ausgebildet sein kann. Insbesondere ist die Analyseeinrichtung nach Art einer Chipkarte ("Lab-on-a-Chip") ausgebildet, die alle Mittel zur Behandlung und Auswertung von Messproben beinhaltet. Eine zum bestimmungsgemäßen Betrieb der Einrichtung notwendige, zugehörige Steuer-/Auslese-Einrichtung ist in diesem Zusammenhang nicht gezeigt.

Im Einzelnen besteht die Einrichtung aus einem kartenartigen Körper 1 aus Kunststoff (Plastik), welcher Ein- und Auslässe für Fluide aufweist. Insbesondere ist ein Einlass 2 ("Port") zum Einbringen von Wasser und ein Einlass 3 ("Port") zum Einbringen einer Messprobe, beispielsweise Blut, vorhanden. Dabei ist wesentlich, dass über geeignete Fluidik-Einrichtungen 2 bis 10, insbesondere Kanäle und Kavitäten unterschiedlicher Geometrie, Messproben und Lösungsmittel transportiert und zusammengeführt werden.

Die Fluidik-Einrichtungen beinhalten im Einzelnen neben den bereits erwähnten Wasser- und Probenports 2, 3 zwei Reagenz-Kanäle 4, 4' sowie einen Strömungskanal 5 mit Auslass 6, ei nen Aufnahmekanal 8 für Abfall und einem weiteren Fluidik-Kanal 9. Mit 10 ist ein zentraler Mischbereich im Strömungskanal 5 für die Probenaufbereitung bezeichnet.

Nach Behandlung der Probe in der Mischstrecke 10 und nach Isolierung der in der Messprobe enthaltenen DNA werden die freigesetzten DNA gesammelt und einer PCR-Kammer 20 zur Durchführung einer PCR (Polymerase Chain Reaction) zugeführt.

Ein Verfahren zur Durchführung der PCR speziell mit Trockenreagenzien in einer Anordnung gemäß Figur 1 wird in der parallelen Anmeldung der Anmelderin mit gleicher Anmeldepriorität und der Bezeichnung "Verfahren und Anordnung zur DNA-Amplifikation mittels PCR unter Einsatz von Trockenreagenzien" im Einzelnen beschrieben.

Neben den Mitteln zur PCR enthält die Karte 1 ("Cartridge") weiterhin ein Detektionsmodul 30 mit Zufluss 32 und Abfluss 31, sowie zugehörige elektrische Anschlüsse für eine Sensorsignalauslesung. Ferner sind Mittel zur Aufnahme von Reagenzien zur Detektion, beispielsweise Kanäle 4 und 4', sowie zur Aufnahme von Abfall, wie Blut und verbrauchte Reagenzlösungen, beispielsweise Kanäle 8 und 9, vorhanden. Es wird somit eine Integration gewährleistet, bei der keine gesundheitsgefährdenden Substanzen nach außen gelangen können.

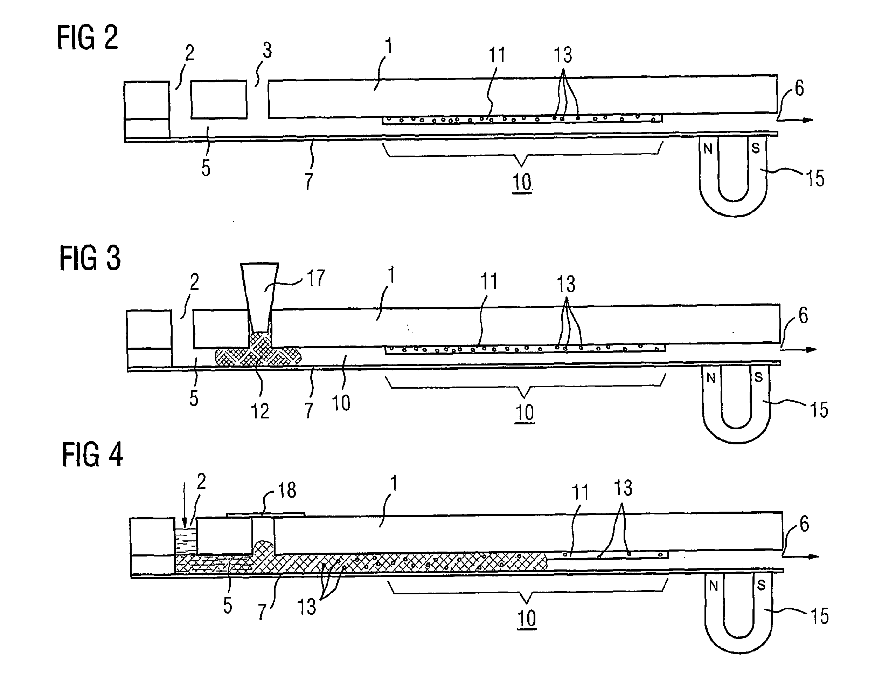

Aus den Figuren 2 bis 6 ist ersichtlich, dass die Plastikkarte 1 aus Figur 1 mit Wasserport 2 und Blutport 3 einen Durchflusskanal 5 beinhaltet, über den Wasser als Spülflüssigkeit und beispielsweise Blut bzw. eine DNA-Lösung bzw. eine Zell- /Bakterien-/Viren-Suspension als Messprobe in das ansonsten geschlossene System eingebracht werden. Über einen Auslass 6 werden die Abfälle in den Abfall-Kanal 8 bzw. in eine Abfall-Kavität überführt.Der Durchflusskanal 5 ist auf der der Plastikkarte 1 gegenüberliegenden Seite durch eine Klebefolie 7 abgedeckt. Im zentralen Bereich des Durchflusskanals 5 wird eine Mischstrecke 10 gebildet, in der speziell zur DNA-Isolierung aus Vollblut die Messproben aufbereitet werden.

Die DNA-Isolierung aus Vollblut erfolgt über einen Aufschluss der weißen Blutzellen. Dazu werden die Zellen chemisch mit einem Lyse-Reagenz aufgebrochen und die darin enthaltenen DNA freigesetzt und gesammelt. Insbesondere bedient man sich dabei der bekannten Vorgehensweise, über so genannte Magnet-Beads, die DNA nach Aufschluss der Zelle zumindest vorübergehend zu binden und durch externe Magnetfelder aufzukonzentrieren.

Speziell aus Figur 2 ist ersichtlich, dass in der Plastikkarte 1 in einem Bereich 10 des Durchflusskanals 5 eine lagerstabile Trockensubstanz 11, die wasserlöslich ist, angeordnet ist. Unter Lagerstabilität ist in diesem Zusammenhang zu verstehen, dass der Feststoff mehrere Monate bei Raumtemperatur gelagert werden kann unter Erhaltung der den Probenaufschluss bewirkenden Eigenschaft.

Die Trockensubstanz 11 weist in den Figuren 2 und 3 eine gute Haftung an den Wänden des Durchflusskanals auf. Dies kann z.B. durch Zugabe eines Filmbildners realisiert werden. Im Einzelnen ist dafür im Bereich der Mischstrecke 10 großflächig ein Gemisch 11 aus einem bekannten Lyse-Reagenz mit Magnet-Beads 13 angeordnet. Weiterhin ist ein Magnet 15 symbolhaft angedeutet, der die magnetische Handhabung zum Einsammeln der Magnet-Beads 13 mit daran gebundener DNA verdeutlicht.

Aus Figur 3 und Figur 4 ist ersichtlich, dass in die Anordnung nach Figur 2 über eine Pipettenspitze 17 Vollblut 12 in das Messsystem eingebracht wird. Nach dem Einbringen der Probe wird der Port 3 mittels einer Klebefolie 18 abgeschlossen.

Über den Port 2 wird Wasser bzw. eine Pufferlösung eingebracht. Wasser, bzw. Puffer-Lösung vermischt sich entlang der Mischstrecke mit der Blutprobe 12, wobei gleichermaßen das Lyse-Reagenz 11 einschließlich der Magnet-Beads 13 aufgelöst bzw. suspendiert werden und zur Wirkung kommen. Dafür werden zunächst die Zellwände der weißen Blutzellen durch das Lyse-Reagenz aufgebrochen und anschließend die dabei freigesetzte DNA an der Oberfläche der Magnet-Beads gebunden.

Entsprechend Figur 4 wird also durch das Wasser/die Puffer-Lösung die Blutprobe verdünnt und gleichzeitig das Lyse-Reagenz angelöst, welches dazu dient, den Aufschluss der weißen Blutzellen zu gewährleisten. Gleichermaßen werden aber auch die an der Wandung des Kanals befindlichen Magnet-Beads des Gemisches 11 in Lösung gebracht bzw. suspendiert, wobei die DNA über diese Magnet-Beads 13 gebunden werden. Somit werden die wesentlichen vier Verfahrensschritte und zwar:
- Probenverdünnung,
- Reagenzauflösung/Magnet-Bead-Suspension,
- Zellaufschluss und
- DNA-Bindung
in einem einzigen Vorgang realisiert

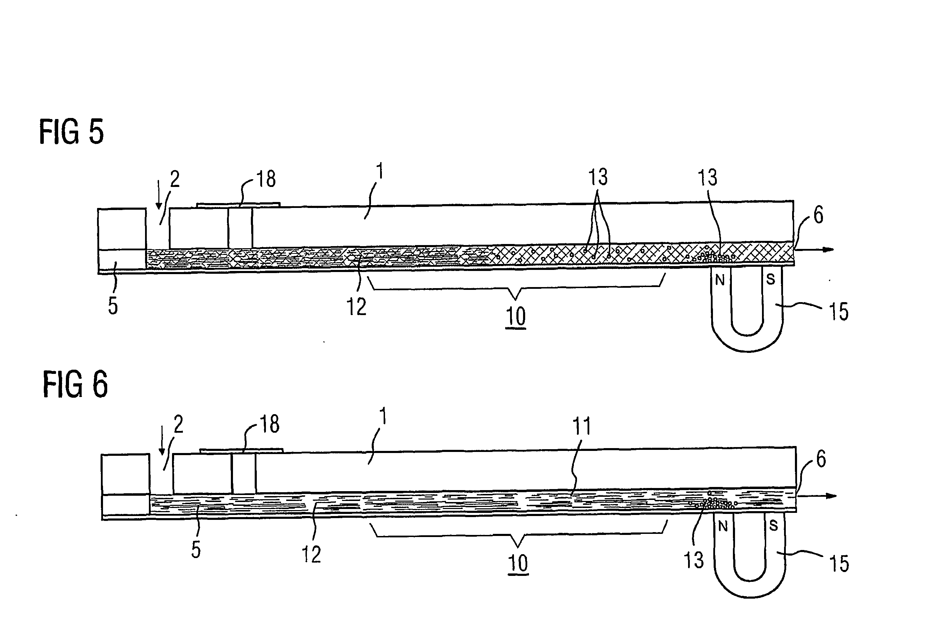

Letzteres wird anhand Figur 5 verdeutlicht. Die über die Magnet-Beads 13 magnetisch handhabbare DNA kann nunmehr gesammelt werden, während PCR-inhibierende Substanzen durch Wasser oder eine Pufferlösung ausgewaschen und über den Auslass 6 zum Abfall gebracht werden.

Als Ergebnis der vorbeschriebenen Maßnahmen ergibt sich entsprechend Figur 6 eine Aufkonzentrierung der über die Magnet-Beads 13 gebundenen DNA an einem der Magnetpole des Magneten 15. Diese DNA kann für eine Analyse verwendet werden, wobei zunächst insbesondere eine PCR durchgeführt wird.

Alternativ zur Verwendung von beweglichen, d.h. suspendierten, Magnet-Beads in Kombination mit einem Magnetfeld als DNA-bindende Mittel können auch immobilisierte, d.h. ortsfeste, DNA-bindende Mittel, am Ausgang des Zellaufschluss-Kanals platziert, eingesetzt werden. In dieser Ausführungsform wird die aus den Zellen freigesetzte DNA beim Verlassen des Zellaufschlusskanals durch Überströmen über das ortsfeste DNA-bindende Mittel dem Flüssigkeitsstrom entzogen und auf dem DNA-bindenden Mittel gebunden. Als DNA-bindende Mittel kommen z.B. Hydrogele mit DNA-bindenden Fängermolekülen oder ähnliches in Frage.

Durch das erfindungsgemäße Verfahren und die zugehörige Anordnung wird insbesondere eine einstufige, einfache und schnelle DNA-Isolierung realisiert, die nur ein Minimum an mikrofluidischen Mitteln, Reagenzien und Verfahrensschritten erforderlich macht.

Die isolierten DNA können anschließend der PCR unterzogen werden. Mit der PCR wird die Konzentration der DNA auf einen analytisch nachweisbaren Wert erhöht. Dabei kann nunmehr die PCR in den Analysevorgang eingebunden werden. Die Analyse erfolgt dann im Detektionsmodul 30 entsprechend Figur 1, auf den hier nicht weiter eingegangen wird.

Zusammenfassend werden bei der Erfindung folgende Maßnahmen verwirklicht:
- Die im Mikrokanal bzw. Mikrokavität eingebrachten Substrate haben DNA-bindende Eigenschaften. Dafür werden z.B. DNA-bindende Magnet-Beads verwendet;
- die Lyse-Reagenzien und Magnet-Beads sind insbesondere zusammen in einer einzigen Trockenmatrix enthalte;
- es ist ein Eingabe-Port für eine Vollblut-Probe Zell-/Bakterien-/Viren-Suspension) vorhanden;
- es sind Mittel zur Zufuhr von Wasser vorhanden. Dies kann z.B. ein Zufluss-Port zum Anschluss an externe Wasserzufuhr sein;
- es sind gegebenenfalls Mittel zur Zudosierung von Salzen, z.B. zur Einstellung einer definierten Ionenstärke, vorhanden;
- es sind gegebenenfalls Mittel (Kanäle und Kavitäten) zur Verdünnung der Blutprobe vorhanden;
- es sind gegebenenfalls weitere Mittel, insbesondere Kanäle
- und Kavitäten, zur Bereitung von definierten Salz-/Pufferlösungen zum Waschen der gebundenen DNA, z.B. DNA-Magnet-bead-Komplex, vorhanden;
- es sind Mittel zum Durchströmen des mit Lyse/Bead-Reagenz beschichteten Mikrokanals bzw. Mikrokavität mit Blut bzw. Blut/Wasser-, Blut/Puffer-Gemisches, vorzugsweise außerhalb der Cartridge, vorhanden;
- es sind Mittel zum Generieren eines Magnetfeldes zum Fixieren des DNA/Magnet-Bead-Komplexes z.B. am Ausgang des Mikrokanals vorhanden, wobei sich diese Mittel vorzugsweise außerhalb der Cartridge befinden.

Somit ist gewährleistet, dass der gesamte Analysevorgang einschließlich der Probenbereitstellung im abgeschlossenen System, das eine Einmal-Cartridge aus umweltverträglichem Kunststoff bildet, erfolgt.

## Patentansprüche

1. Verfahren zur DNA-Isolierung unter Entfernung von eine nachfolgende PCR störenden Bestandteilen, bei dem
- eine vollständige Integration aller Stoffe und Verfahrensschritte in einer einmal verwendbaren Einheit (sog. Cartridge), die einen Zutritt einer Probe mit DNA erlaubt, erfolgt
- zur Freisetzung der DNA Trockenreagenzien verwendet werden,
- DNA-bindende Substrate zur Isolation der freigesetzten DNA eingesetzt werden,
- die isolierten DNA gesammelt und zum Ort der nachfolgenden PCR transportiert werden,
wobei ein Aufschluss von DNA enthaltenden biologischen Behältnissen, insbesondere Zellen, Bakterien oder Viren, erfolgt, wozu die zur Ausführung des Aufschlusses und der weiteren Verfahrensschritte notwendigen Reagenzien in einer bei Raumtemperatur stabilen Form in der Einheit (Cartridge)gelagert sind, **dadurch gekennzeichnet,**
- **dass** zum Aufschluss der die DNA enthaltenden biologischen Behältnisse eine in einem Aufschlusskanal an einer Wandung befindliche Trockenmatrix aus Lyse-Reagenzien und den DNA-bindenden Substraten mit den biologischen Behältnissen, insbesondere Zellen, Bakterien oder Viren, in Kontakt gebracht wird, wobei die DNA bindenden Substrate suspendiert werden
- **dass** die durch den Kontakt mit den Lyse-Reagenzien freigesetzten DNA unmittelbar, gleichzeitig an die DNA bindenden Substrate gebunden, und
- die Substrate mit den daran gebundenen DNA gesammelt und zum Ort der PCR transportiert werden.

2. Verfahren nach Anspruch 1, **dadurch gekennzeichnet, dass** als DNA enthaltende Behältnisse weiße Blutzellen verwendet werden.

3. Verfahren nach Anspruch 2, **dadurch gekennzeichnet, dass** das trockengelagerte und lagerstabile Lyse-Reagenz unter Zuhilfenahme von Wasser mit den weißen Blutzellen in Kontakt gebracht wird.

4. Verfahren nach Anspruch 1, **dadurch gekennzeichnet, dass** eine Verdünnung der Probe, Auflösen bzw. Suspendieren des Trockenreagenz, Aufschluss der biologischen Behältnisse und Bindung der DNA in einem einzigen Verfahrensschritt erfolgen.

5. Verfahren nach Anspruch 1, **dadurch gekennzeichnet, dass** als DNA-bindendes Substrat sog. Magnet-Beads verwendet werden.

6. Verfahren nach Anspruch 1, **dadurch gekennzeichnet, dass** als DNA-bindendes Substrat ein am Ausgang des Zellaufschluss-kanals immobilisiertes Hydrogel mit DNA-Fängersubstanzen verwendet wird.

7. Verfahren nach Anspruch 6, **dadurch gekennzeichnet, dass** die freigesetzten DNA durch die Magnet-Beads gebunden und zu einem Sammel- bzw. Reaktionsort befördert wird.

8. Verfahren nach Anspruch 5, **dadurch gekennzeichnet, dass** die Magnet-Beads durch Applikation eines Magnetfeldes eingesammelt werden.

9. Verfahren nach Anspruch 8, **dadurch gekennzeichnet, dass** strömende Magnet-Beads durch ein ortsfestes Magnetfeld eingesammelt werden.

10. Verfahren nach Anspruch 1, **dadurch gekennzeichnet, dass** am Reaktionsort die PCR durch Thermozyklisierung mit trockengelagerten, wasserlöslichen Reagenzien durchgeführt wird.

11. Verfahren nach Anspruch 1, **dadurch gekennzeichnet, dass** eine in die Einheit eingegebene Blutprobe verdünnt und durch den Reagenzkanal zum Reaktionskanal für die PCR gepumpt wird.

12. Verfahren nach Anspruch 11, **dadurch gekennzeichnet, dass** nach der DNA-Isolierung Restblut, Blutbestandteile und der Reagenzienabfall in der Einheit verbleiben, so dass kein direkter Kontakt mit gesundheitsgefährdenden Stoffen erfolgen kann.

13. Verfahren nach einem der vorhergehenden Ansprüche, **dadurch gekennzeichnet, dass** als Einheit zur Ausführung der Reaktionen ein Einmalprodukt verwendet wird, das in Massenfertigung klein und kostengünstig herzustellen ist.

14. Anordnung zur Durchführung des Verfahrens nach Anspruch 1 oder einem der Ansprüche 2 bis 13, mit einer Einheit zur Aufnahme von Proben und/oder Reagenzien, wobei wenigstens ein Mikrokanal (5) zur Aufnahme von Reagenzien vorhanden ist, in dem eine Trockenmatrix aus Lyse-Reagenz und DNA-bindendes Substrat im Mikrokanal (5) als Gemisch mit vernachlässigbarem Dampfdruck, das bei Raumtemperatur eine stabile Substanz bildet, vorliegt und dass das Gemisch aus Lyse-Reagenz und DNA-bindendes Substrat durch Zusatz eines Filmbildners großflächig als Schicht (11) an der Wandung des Mikrokanals (5) gebunden ist.

15. Anordnung nach Anspruch 14, **dadurch gekennzeichnet, dass** die DNA enthaltenden biologischen Behältnisse insbesondere Zellen, Bakterien, Viren sind.

16. Anordnung nach Anspruch 15, **dadurch gekennzeichnet, dass** zur Durchführung des Aufschlusses eines biologischen Behältnisses die trockene Substanz mit vernachlässigbarem Dampfdruck im Mikrokanal (5) ein Lyse-Reagenz ist, welches spezifische Eigenschaften für weiße Blutzellen hat.

17. Anordnung nach Anspruch 16, **dadurch gekennzeichnet, dass** der Kanal (5) für die Lyse-Substanz in einer PCR-Kavität (20) mündet.

18. Anordnung nach Anspruch 14, **dadurch gekennzeichnet, dass** die DNA-bindenden Substrate sog. Magnet-Beads (10) sind.

19. Anordnung nach einem der Ansprüche 14 bis 18, **dadurch gekennzeichnet, dass** ein Eingabeport (3) für Vollblut-Proben vorhanden ist.

20. Anordnung nach einem der Ansprüche 14 bis 19, **dadurch gekennzeichnet, dass** Mittel (2) zur Zufuhr von Wasser vorhanden sind.

21. Anordnung nach einem der Ansprüche 14 bis 20, **dadurch gekennzeichnet, dass** trockene Puffersubstanzen zur Einstellung einer definierten Ionenstärke vorhanden sind.

22. Anordnung nach einem der Ansprüche 14 bis 21, **dadurch gekennzeichnet, dass** Mittel zum Mischen von Blutprobe und Wasser, bzw. einer Pufferlösung, vorhanden sind.

23. Anordnung nach einem der Ansprüche 14 bis 22, **dadurch gekennzeichnet, dass** Mittel zum Durchströmen des mit Lyse-/ Bead-Reagens beschichteten Mikrokanals (5) bzw. Mikrokavität (20) mit Blut, Blut/Wasser- oder Blut/Puffer-Gemische vorhanden sind.

24. Anordnung nach einem der Ansprüche 14 bis 23, **dadurch gekennzeichnet, dass** Mittel zum Generieren eines Magnetfeldes zwecks Fixieren eines DNA-/Magnet-Bead-Komplexes in der PCR-Kavität (20) vorhanden sind.

25. Anordnung nach einem der Ansprüche 14 bis 24, **dadurch gekennzeichnet, dass** Mittel zur Thermozyklisierung vorhanden sind.

## Claims

1. Method for DNA isolation while removing constituents that interfere with a subsequent PCR, in which
- all the materials and method steps are fully integrated in a closed analysis unit having a single-use unit (so-called cartridge) which allows a sample with DNA to enter;
- dry reagents are used to release the DNA;
- DNA-binding substrates are used to isolate the released DNA;
- the isolated DNAs are collected and transported to the site of the subsequent PCR,
disintegration of biological structures containing DNA being carried out, particularly cells, bacteria or viruses, to which end the reagents necessary for carrying out the disintegration and the further method steps are stored in the unit (cartridge) in a form that is stable at room temperature,
**characterized in that**
- a dry matrix comprising lysis reagents and the DNA-binding substrates, which lies on a wall in a disintegration channel, is brought in contact with biological structures, particularly cells, bacteria or viruses, in order to disintegrate the biological structures containing the DNA, the DNA-binding substrates being suspended,
- **in that** the DNAs released by contact with the lysis reagents are bound directly, simultaneously to the DNA-binding substrates, and
- the substrates with the DNAs bound thereon are collected and transported to the site of the PCR.

2. Method according to Claim 1, **characterized in that** white blood cells are used as structures containing DNA.

3. Method according to Claim 2, **characterized in that** the dryly stored and storage-stable lysis reagent is brought in contact with the white blood cells with the aid of water.

4. Method according to Claim 1, **characterized in that** diluting of the sample, dissolving or suspending of the dry reagent, disintegrating of the biological structures and binding of the DNA take place in a single method step.

5. Method according to Claim 1, **characterized in that** so-called magnet beads are used as the DNA-binding substrate.

6. Method according to Claim 1, **characterized in that** a hydrogel with DNA capture substances, which is immobilized at the exit of the cell disintegration channel, is used as the DNA binding substrate.

7. Method according to Claim 6, **characterized in that** the released DNA is bound and transported by the magnet beads to a collection or reaction site.

8. Method according to Claim 5, **characterized in that** the magnet beads are concentrated by applying a magnetic field.

9. Method according to Claim 8, **characterized in that** flowing magnet beads are concentrated by a static magnetic field.

10. Method according to Claim 1, **characterized in that** the PCR is carried out by thermocycling with dryly stored watersoluble reagents at the reaction site.

11. Method according to Claim 1, **characterized in that** the blood sample put into the unit is diluted and pumped through the reagent channel to the reaction channel for the PCR.

12. Method according to Claim 11, **characterized in that** residual blood, blood constituents and the reagent waste remain in the unit after the DNA isolation, so that no direct contact can take place with substances hazardous to health.

13. Method according to one of the preceding claims, **characterized in that** a disposable product which is small and inexpensive to make in mass production is used as the unit for performing the reactions.

14. Assembly for carrying out the method according to Claim 1 or one of Claims 2 to 13,
having a unit for receiving samples and/or reagents, at least one microchannel (5) being provided for receiving reagents, in which a dry matrix comprising lysis reagent and DNA-binding substrate is provided in the microchannel (5) as a mixture with a negligible vapor pressure, which forms a stable substance at room temperature, and in that the mixture of lysis reagent and the DNA-binding substrate is bound as a layer (11) over a large area on the wall of the microchannel (5) by adding a film-forming agent.

15. Assembly according to Claim 14, **characterized in that** the biological structures containing DNA are in particular cells, bacteria, viruses.

16. Assembly according to Claim 15, **characterized in that** for carrying out the disintegration of a biological structure, the dry substance with a negligible vapor pressure in the microchannel (5) is a lysis reagent which has specific properties for white blood cells.

17. Assembly according to Claim 16, **characterized in that** the channel (5) for the lysis substance opens in a PCR cavity (20).

18. Assembly according to Claim 14, **characterized in that** the DNA-binding substrates are so-called magnet beads (10).

19. Assembly according to one of Claims 14 to 18,
**characterized in that** an input port (3) for whole blood samples is provided.

20. Assembly according to one of Claims 14 to 19,
**characterized in that** means (2) for supplying water are provided.

21. Assembly according to one of Claims 14 to 20,
**characterized in that** dry buffer substances are provided for adjusting a defined ionic strength.

22. Assembly according to one of Claims 14 to 21,
**characterized in that** means are provided for mixing a blood sample and water, or a buffer solution.

23. Assembly according to one of Claims 14 to 22,
**characterized in that** means are provided so that blood, blood/water or blood/buffer mixtures can flow through the microchannel (5) or microcavity (20) coated with lysis/bead reagent.

24. Assembly according to one of Claims 14 to 23,
**characterized in that** means (25) are provided for generating a magnetic field for the purpose of fixing a DNA-magnet bead complex in the PCR cavity (20).

25. Assembly according to one of Claims 14 to 24,
**characterized in that** thermocycling means are provided.

## Revendications

1. Procédé d'isolement d'ADN en éliminant des constituants gênants une PCR venant ensuite, dans lequel
- on effectue une intégration complète de toutes les substances et stades de procédé dans une unité pouvant être utilisée une fois ( ce que l'on appelle une cartridge ), qui permet un accès à un échantillon ayant de l'ADN,
- on utilise des réactifs secs pour libérer l'ADN,
- on utilise des substrats fixant l'ADN pour l'isolement de l'ADN libéré,
- on recueille l'ADN isolé et on le transporte à l'emplacement de la PCR venant ensuite,
dans lequel on effectue une attaque de réceptacles biologiques contenant de l'ADN, notamment des cellules, des bactéries ou de virus, en stockant les réactifs nécessaires à la réalisation de l'attaque et des autres stades du procédé sous une forme stable à la température ambiante dans l'unité ( cartridge ), **caractérisé**
- **en ce que**, pour l'attaque des réceptacles biologiques contenant l'ADN, on met une matrice sèche se trouvant sur une paroi dans un canal d'attaque et composée de réactifs de lyse, et des substrats fixant l'ADN en contact avec les réceptacles biologiques, notamment des cellules, des bactéries et des virus, les substrats fixant l'ADN étant mis en suspension,
- **en ce que** l'on fixe les ADN libérés par le contact avec les réactifs de lyse en même temps directement sur les substrats fixant l'ADN et
- on recueille les substrats ayant les ADN qui sont fixés et on les transporte à l'emplacement de la PCR.

2. Procédé suivant la revendication 1, **caractérisé en ce que** l'on utilise des globules blancs comme réceptacles contenant de l'ADN.

3. Procédé suivant la revendication 2, **caractérisé en ce que** l'on met le réactif de lyse stocké à sec et stable au stockage en contact avec les globules blancs en s'aidant d'eau.

4. Procédé suivant la revendication 1, **caractérisé en ce que** l'on effectue une dilution de l'échantillon, une dissolution ou une mise en suspension du réactif sec, une attaque des réceptacles biologiques et une fixation de l'ADN en un stade de procédé unique.

5. Procédé suivant la revendication 1, **caractérisé en ce que** l'on utilise ce que l'on appelle des perles magnétiques comme substrat fixant l'ADN.

6. Procédé suivant la revendication 1, **caractérisé en ce que** l'on utilise comme substrat fixant l'ADN un hydrogel immobilisé à la sortie du canal d'attaque des cellules et ayant des substances de capture de l'ADN.

7. Procédé suivant la revendication 6, **caractérisé en ce que** l'on fixe les ADN libérés par des perles magnétiques et on les transporte à un emplacement de collecte et de réaction.

8. Procédé suivant la revendication 5, **caractérisé en ce que** l'on collecte les perles magnétiques en appliquant un champ magnétique.

9. Procédé suivant la revendication 8, **caractérisé en ce que** l'on collecte les perles magnétiques en écoulement par un champ magnétique fixe.

10. Procédé suivant la revendication 1, **caractérisé en ce que** l'on effectue à l'emplacement de réaction la PCR par thermocyclisation avec des réactifs stockés à sec et solubles dans l'eau.

11. Procédé suivant la revendication 1, **caractérisé en ce que** l'on dilue un échantillon de sang chargé dans l'unité et on le pompe par le canal de réactifs vers le canal de réaction pour la PCR.

12. Procédé suivant la revendication 11, **caractérisé en ce qu'**après l'isolement de l'ADN il subsiste du sang restant, des constituants du sang et le déchet des réactifs dans l'unité, de sorte qu'un contact direct avec des substances nocives ne peut pas s'effectuer.

13. Procédé suivant l'une des revendications précédentes, **caractérisé en ce que** l'on utilise comme unité pour réaliser les réactions un produit à jeter, qui peut être fabriqué d'une manière peu coûteuse et en étant petit dans une fabrication de masse.

14. Dispositif pour la mise en oeuvre du procédé suivant la revendication 1 ou l'une des revendications 2 à 13, comprenant une unité de réception d'échantillons et/ou de réactifs, au moins un microcanal (5) étant prévu pour la réception de réactifs, dans lequel il y a une matrice (7) composée de réactif de lyse et de substrat fixant l'ADN dans le microcanal (5) sous la forme d'un mélange ayant une tension de vapeur négligeable qui forme une substance stable à la température ambiante et en ce que le mélange composé du réactif de lyse et du substrat fixant l'ADN est fixé à la paroi du microcanal (5) sous la forme d'une couche (11) sur une grande surface par addition d'un agent filmogène.

15. Dispositif suivant la revendication 14,
**caractérisé en ce que** les réceptacles biologiques contenant de l'ADN sont, notamment, des cellules, des bactéries, des virus.

16. Dispositif suivant la revendication 15,
**caractérisé en ce que**, pour effectuer l'attaque d'un réceptacle biologique, la substance (16) ayant une tension de vapeur négligeable dans le microcanal (5) est un réactif de lyse qui a des propriétés spécifiques pour des globules blancs.

17. Dispositif suivant la revendication 16,
**caractérisé en ce que** le canal (5) pour la substance de lyse débouche dans une cavité PCR (20).

18. Dispositif suivant la revendication 14,
**caractérisé en ce que** les substrats fixant l'ADN sont ce que l'on appelle des perles (10) magnétiques.

19. Dispositif suivant l'une des revendications 14 à 18, **caractérisé en ce qu'**il est prévu un orifice (3) d'entrée d'échantillons de sang complet.

20. Dispositif suivant l'une des revendications 14 à 19, **caractérisé en ce qu'**il est prévu des moyens (2) d'apport d'eau.

21. Dispositif suivant l'une des revendications 14 à 20, **caractérisé en ce que** des substances tampons sèches sont prévues pour régler une force ionique définie.

22. Dispositif suivant l'une des revendications 14 à 21, **caractérisé en ce qu'**il est prévu des moyens de mélange d'échantillon de sang et d'eau ou d'une solution tampon.

23. Dispositif suivant l'une des revendications 14 à 22, **caractérisé en ce qu'**il est prévu des moyens pour faire passer du sang, des mélanges de sang et d'eau ou des mélanges de sang et de tampon dans le microcanal (5) revêtu de réactifs de lyse/perle ou dans la microcavité (20).

24. Dispositif suivant l'une des revendications 14 à 23, **caractérisé en ce qu'**il est prévu des moyens pour produire un champ magnétique en vue d'une immobilisation d'un complexe ADN/perle magnétique dans la cavité (20) PCR.

25. Dispositif suivant l'une des revendications 14 à 24, **caractérisé en ce qu'**il est prévu des moyens de thermocyclisation.
